# EUROPÄISCHE PATENTSCHRIFT

(11) **EP 3 530 302 B1**
(45) Veröffentlichungstag und Bekanntmachung des Hinweises auf die Patenterteilung: **30.07.2025**
(21) Anmeldenummer: 19155913.7
(22) Anmeldetag: 07.02.2019
(51) Int. Cl.: A61M 1/36, A61M 1/34, A61M 1/38, B01D 63/02, B01D 69/08, B01D 71/56, B01D 71/78

(54) **VORRICHTUNG ZUR ENTFERNUNG VON NOXEN AUS BLUT, EXTRAKORPORALES PERFUSIONSSYSTEM MIT EINER SOLCHEN VORRICHTUNG SOWIE VERFAHREN ZUR HERSTELLUNG EINER SOLCHEN VORRICHTUNG**
DEVICE FOR THE REMOVAL OF NOXIOUS AGENTS FROM BLOOD, EXTRACORPOREAL PERFUSION SYSTEM COMPRISING SUCH A DEVICE AND METHOD FOR PRODUCING SUCH A DEVICE
DISPOSITIF D'ÉLIMINATION DES NOXES DU SANG, SYSTÈME EXTRACORPOREL DE PERFUSION DOTÉ D'UN TEL DISPOSITIF ET PROCÉDÉ DE FABRICATION D'UN TEL DISPOSITIF

(30) Priorität: 23.02.2018 DE 102018104177
(43) Veröffentlichungstag der Anmeldung: 28.08.2019
(73) Patentinhaber: B. Braun Avitum AG, 34212 Melsungen (DE)
(72) Erfinder: MANDRY, Peter, 01326 Dresden (DE)
(74) Vertreter: Winter, Brandl - Partnerschaft mbB

(56) Entgegenhaltungen:
- EP-A1- 1 602 387
- EP-B1- 1 602 387
- EP-B1- 1 776 175
- WO-A1-2011/015197

## Beschreibung

Die Erfindung betrifft eine Vorrichtung zur Entfernung von, insbesondere negativ geladenen, Noxen aus Blut, welches Plasma und zelluläre Bestandteile aufweist, in einem extrakorporalen Perfusionssystem, mit einem Gehäuse und einer Vielzahl von innerhalb des Gehäuses vorgesehenen Hohlfasern, welche konfiguriert sind, mit dem Blut durchströmt zu werden, wobei die Hohlfasern jeweils eine Vielzahl von Poren aufweisen, welche derart ausgebildet sind, dass das Plasma des Bluts durch die Poren von einer Innenseite der Hohlfasern zu einer Außenseite der Hohlfasern strömen kann, wobei die Hohlfasern derart, insbesondere chemisch, modifiziert bzw. vorbehandelt sind, dass sie eine funktionalisierte, die Noxen an sich bindende und aus dem Blut entfernende Oberfläche aufweisen. Weiterhin betrifft die Erfindung ein extrakorporales Perfusionssystem mit einer solchen Vorrichtung sowie ein Verfahren zur Herstellung einer solchen Vorrichtung.

### Stand der Technik

Eine Sepsis bzw. Blutvergiftung ist eine komplexe systemische Entzündungsreaktion des menschlichen Organismus, welche auf eine Infektion durch Bakterien, deren Toxine oder Pilze zurückzuführen ist. Bei vielen Patienten endet auch heute noch trotz aller therapeutischen Maßnahmen eine schwere Sepsis bzw. ein septischer Schock tödlich. Gründe für das Auftreten einer Sepsis liegen unter anderem in der Anwendung von Kathetern und Endoskopen, in der Implantation von Prothesen, in chirurgischen Eingriffen, im Einsatz von immunsuppressiven Medikamenten, in der Zunahme an älteren Patienten und in der steigenden Resistenz von Bakterien gegenüber Antibiotika. Infektionen von Patienten werden heutzutage häufig durch (multi-)resistente Bakterien verursacht.

Aus dem Stand der Technik schon länger bekannte Verfahren wie etwa die Plasmapherese, auch Plasmaaustauschbehandlung genannt, oder Antikörper-Therapieverfahren haben die Prognose für einen septischen Patienten nicht wesentlich verbessern können. Insbesondere hat sich die Plasmapherese als unselektiv und ineffizient herausgestellt, da dem Patienten neben Toxinen und proinflammatorischen Cytokinen auch protektive, anti-intiammatorische Mediatoren entzogen werden. Weiterhin benötigt ein einzelner Therapiezyklus ein Austauschvolumen von etwa 12 Litern an Plasma (etwa 50 Spender), was ein zusätzliches Risiko von Infektionen oder allergischen Reaktionen mit sich bringt. Antikörper-Therapieverfahren sind aufgrund der technisch aufwändigen Gewinnung, Reinigung und Charakterisierung der jeweiligen Antikörper sehr teuer und es besteht bei deren Anwendung unter anderem die Gefahr einer allergischen Gegenreaktion des Körpers auf die Antikörper.

Weiterhin sind aus dem Stand der Technik auch Behandlungen von Blut oder Plasma in einem extrakorporalen Perfusionssystem zur Neutralisation bzw. Elimination pathogener Blutbestandteile wie etwa Lipopolysacchariden, Lipoteichonsäuren etc., unter Verwendung von geeigneten Adsorbermaterialien bekannt.

Beispielsweise sind in der US 4,576,928 oder der DE 39 32 971 poröse Trägermaterialien mit immobilisiertem Polymyxin B beschrieben, welche sich jedoch in der klinischen Anwendung als ungeeignet herausgestellt haben, da der Ligand Polymyxin B schwere nephrotoxische und neurotoxische Schäden bei seiner Freisetzung in der Blutbahn hervorruft.

In der DE 41 13 602 A1 sind polyethylenimin-modifizierte Percellulosen als Adsorber offenbart, welche jedoch eine geringe Bindungskapazität für Lipopolysaccharide besitzen, so dass bei ihrer Verwendung in einem extrakorporalen Perfusionssystem das medizinisch tolerierbare extrakorporale Totvolumen überschritten wird.

In der DE 44 35 612 A1 ist weiterhin ein Plasmaperfusionsverfahren beschrieben, welches sich für eine Elimination von Lipopolysacchariden und TNF-α (Tumornekrosefaktor-α) eignet. Dieses Verfahren ist jedoch sehr komplex, hämodynamisch nachteilhaft, da es ein sehr großes extrakorporales Totvolumen erfordert, und eliminiert zudem neben Lipopolysacchariden und TNF-α auch das für die plasmatische Gerinnung essentielle Fibrinogen, so dass eine Anwendung dieses Verfahrens je nach Ausgangskonzentration von Fibrinogen auf lediglich zwei bis drei konsekutive Behandlungen beschränkt ist, was für eine effektive Behandlung des Patienten zumeist ungenügend ist.

Eine besonders effektive Entfernung von negativ geladenen Noxen aus dem Plasma des Bluts ist in der EP 1 602 387 A1 offenbart. In der in dieser Veröffentlichung offenbarten Vorrichtung sind Hohlfasern vorgesehen, welche chemisch derart modifiziert sind, dass die geladenen Lipopolysaccharide (LPS) und Lymphotoxine-α (LTA) besonders gut an diese gebunden und somit aus dem Plasma entfernt werden können. Dabei werden die Hohlfasern an der Oberfläche chemisch modifiziert, bevorzugt durch eine Pfropfpolymerisation. Bei der Pfropfpolymerisation werden auf das Hohlfasermaterial Verbindungen, beispielsweise Anionenaustauscher(gruppierungen), mit guter Bindefähigkeit für LPS und LTA aufgepfropft. Als Anionenaustauscher sind dabei als Tentakeln ausgestaltete, längere Ketten mit einer Vielzahl von kationischen Gruppen vorgesehen. Derartige tentakelartige Fortsätze auf dem Hohlfasergrundmaterial sind fähig, mehrere LPS- bzw. LTA-Moleküle zu binden, wodurch eine Effizienzsteigerung der Hohlfasern erreicht werden kann. Für die Modifikation der Hohlfasern mittels Tentakeln werden dabei bevorzugt synthetische, halbsynthetische oder natürliche Polykationenketten eingesetzt, welche in linearer oder verzweigter Form vorliegen können. Bevorzugt sind die Hohlfasern dabei durch (Poly-) Kationenketten, welche tertiäre bzw. quartäre Amine aufweisen, modifiziert.

Die in der EP 1 602 387 A1 offenbarte Vorrichtung weist jedoch den Nachteil auf, dass die chemisch modifizierte, beschichtete bzw. aufgepfropfte Oberfläche inkompatibel mit Blutzellen/ den zellulären Bestandteilen des Bluts ist, so dass vor der Behandlung die Blutzellen durch eine Plasmaseparation von dem Blutplasma getrennt werden müssen. Handelsübliche Plasmaseparatoren bestehen aus Hohlfaserkapillaren mit einer Porengröße von 0,1 bis 0,5 µm. Sie werden über einen maximalen Zeitraum von 4 bis 6 Stunden eingesetzt. Da ein an einer Sepsis leidender Patient über einen Zeitraum von mindestens 74 Stunden behandelt wird, muss der Plasmaseparator daher in diesem Zeitraum sehr häufig gewechselt werden.

Die EP 1 776 175 B1 offenbart ein kontinuierliches Verfahren zur Herstellung einer regioselektiven, porösen Hohlfasermembran, wobei die dabei erzeugte Hohlfasermembran eine Bluttrennung und Blutreinigung in einem Schritt ermöglicht. Die Hohlfasermembran ist dabei grundsätzlich aus einem blutverträglichen Polymer hergestellt und schädigt somit die zellulären Bestandteile des Bluts nicht. Über eine spezielle Plasmabehandlung werden lediglich die Außenseite der Hohlfasermembran und die Poren mit funktionellen Gruppen ausgestattet. Wird Blut schließlich mit Hochdruck durch die Hohlfasern geleitet, dringt lediglich (Blut-)Plasma in die feinen Poren ein. Die zellulären Blutbestandteile sind zu groß und verbleiben in dem blutverträglichen Hauptkanal. In den feinen Poren und an der Außenwand der Hohlfasern fischen schließlich über eine nasschemische Behandlung aufgepfropfte Bindungsmoleküle die Giftstoffe aus der Flüssigkeit.

Das in der EP 1 776 175 B1 offenbarte Verfahren hat jedoch den Nachteil, dass dieses für die Plasmavorbehandlung der Hohlfasermembran/ der Hohlfasern ein komplexes Vakuumsystem mit einer Vielzahl von Vakuumkammern benötigt und somit eine Herstellung der darin offenbarten Hohlfasermembran sehr aufwändig ist.

Die WO 2011/015197 A1 offenbart eine Vorrichtung zur Entfernung von Noxen aus Blut. In der WO 2011/015197 A1 ist es unerwünscht, dass Blutbestandteile von der Blutseite zur Gasseite der Hohlfasern übergehen. Entweder die Innenseiten oder die Außenseiten der Hohlfasern sind gleichzeitig mit Noxen-bindenden Substanzen und mit Heparin beschichtet.

### Kurzbeschreibung der Erfindung

Vor diesem Hintergrund ist es Aufgabe der vorliegenden Erfindung, die Nachteile aus dem Stand der Technik zu vermeiden oder wenigstens zu mildern und insbesondere eine Bluttrennung und Blutreinigung in einem Schritt (ohne vorherige Plasmaseparation) mit gegenüber dem Stand der Technik in einfacherer Weise hergestellten porösen Hohlfasern/ mit einer gegenüber dem Stand der Technik in einfacherer Weise hergestellten Vorrichtung zur Entfernung von Noxen aus Blut bereitzustellen. Insbesondere soll ein einfaches Behandlungssystem mit langer Anwendungsdauer bereitgestellt werden.

Diese Aufgabe wird durch eine Vorrichtung zur Entfernung von Noxen aus Blut mit den Merkmalen des Anspruchs 1, ein extrakorporales Perfusionssystem mit den Merkmalen des Anspruchs 8 und ein Verfahren zur Herstellung einer Vorrichtung zur Entfernung von Noxen aus Blut mit den Merkmalen des Anspruchs 9 gelöst. Vorteilhafte Ausführungsformen und Weiterbildungen sind in den Unteransprüchen beansprucht und/oder nachfolgend erläutert.

Die Erfindung betrifft zunächst eine Vorrichtung zur Entfernung von negativ geladenen Noxen aus Blut, welches Plasma und zelluläre Bestandteile aufweist, in/ für/ zur Verwendung in einem extrakorporalen Perfusionssystem, mit einem Gehäuse und einer Vielzahl von innerhalb des Gehäuses vorgesehenen Hohlfasern/ Hohlfaserkapillaren, welche konfiguriert sind, mit dem Blut durchströmt zu werden, wobei die Hohlfasern jeweils eine Vielzahl von Poren aufweisen, welche derart ausgebildet sind, dass das Plasma des Bluts durch die Poren von einer Innenseite der Hohlfasern zu einer Außenseite der Hohlfasern strömen kann, wobei die Hohlfasern derart chemisch modifiziert bzw. vorbehandelt sind, dass sie eine funktionalisierte, die negativ geladenen Noxen an sich bindende und aus dem Blut entfernende positiv geladene Oberfläche aufweisen, wobei ausschließlich eine Innenseitenoberfläche der Hohlfasern weiterhin (vollständig/ die gesamte Innenseitenoberfläche) zur Vermeidung/Verhinderung einer Schädigung der zellulären Bestandteile des Bluts beim Strömen des Bluts durch die Hohlfasern mit einer hämokompatiblen und gerinnungshemmenden Abdeckung/Beschichtung versehen ist, so dass lediglich die Poren und eine Außenseitenoberfläche der Hohlfasern die funktionalisierte Oberfläche aufweisen und an der Innenseitenoberfläche der Hohlfasern keine funktionalisierte Oberfläche mehr vorliegt.

Unter einer Noxe im Kontext der vorliegenden Anmeldung ist ein Stoff bzw. eine Substanz zu verstehen, welche/r in nicht erwünschter Weise im Blut eines Lebewesens, beispielsweise eines Menschen, enthalten ist und eine schädigende, pathogene und/oder gefährdende Wirkung auf den Organismus oder auf ein Körperorgan ausübt. Unter Noxen können beispielsweise Lipopolysaccharide (LPS, Endotoxine), Lipoteichonsäuren (LTA), Viren, DNA, etc. verstanden werden. Unter einem extrakorporalen Perfusionssystem ist ein Durchblutungssystem außerhalb des Körpers des Lebewesens zu verstehen. Wird im Kontext der vorliegenden Anmeldung von Blut gesprochen, ist dabei grundsätzlich eine Suspension aus Plasma und zellulären Bestandteilen wie etwa Erythrozyten, Leukozyten, Thrombozyten, etc. zu verstehen.

Die erfindungsgemäße Vorrichtung ist eingerichtet, sowohl von den zellulären Bestandteilen als auch von dem Plasma des Bluts durchströmt zu werden, so dass keine Separation des Plasmas von den zellulären Bestandteilen vor dem Durchströmen der Vorrichtung mit Blut vonnöten ist. Es ist somit erfindungsgemäß keine Plasmaseparation und somit kein häufiger Wechsel eines Plasmaseparators erforderlich. Die Vorrichtung der vorliegenden Erfindung wird zur Behandlung von Patienten mit Erkrankungen, welche durch eine Invasion von gram-negativen und/oder gram-positiven Bakterien oder anderen negativ geladenen Noxen wie Shigatoxin hervorgerufen werden, verwendet.

Hinsichtlich des Materials der porösen/ Poren aufweisenden Hohlfasern und hinsichtlich der Modifizierung/ Vorbehandlung der Hohlfasern wird vollumfänglich auf die EP 1 602 387 A1 Bezug genommen. Die wichtigsten Aspekte werden nachfolgend jedoch auch in der vorliegenden Anmeldung kurz umrissen.

Grundsätzlich können Hohlfasermaterialien verwendet werden, welche aus Polyamid, Polysulfon, Polyether, Polyethylen, Polypropylen, Polyester oder Derivaten und/oder Mischungen solcher Polymere hergestellt werden. Besonders bevorzugt bestehen die Hohlfasern aus Nylon (Polyamid 66). Diese Membrangrundmaterialien können nach an sich bekannten Methoden, bevorzugt durch Pfropfpolymerisation, modifiziert werden, damit sie eine funktionalisierte, die Noxen an sich bindende und aus dem Blut entfernende Oberfläche erhalten. Eine funktionalisierte Oberfläche zeichnet sich im Kontext der vorliegenden Anmeldung dadurch aus, dass sie eine große Oberfläche und funktionelle, die Noxen anziehende, Gruppen aufweist und somit sowohl eine mechanische als auch eine spezifische Adhäsion der Noxen an den Hohlfasern fördert. Die in der erfindungsgemäßen Vorrichtung eingesetzten Hohlfasern sind derart chemisch modifiziert, dass sich negativ geladene Noxen wie LPS- oder LTA-Moleküle besonders gut an das Hohlfasermaterial binden können und somit aus dem Blut entfernt werden (Hohlfasern mit positiver Ladung).

Eine chemische Modifikation des Hohlfasermaterials erfolgt somit bevorzugt durch Pfropfpolymerisation, bei welcher auf das Hohlfasermaterial Verbindungen aufgepfropft werden, welche eine gute Bindefähigkeit für insbesondere LPS und/oder LTA zeigen.

Dabei hat es sich als besonders vorteilhaft herausgestellt, Anionenaustauschergruppierungen aufzupfropfen. Derartige Anionenaustauschergruppierungen sind bevorzugt als längere Ketten mit einer Vielzahl von kationischen Gruppen, als sogenannte Tentakel, ausgestaltet. Derartige tentakelartige Fortsätze auf dem Grundmaterial sind fähig, mehrere LPS- bzw. LTA-Moleküle zu binden. Für die Modifikation des Hohlfasermaterials mittels Tentakeln werden vorzugsweise synthetische und/oder halbsynthetische und/oder natürliche Polykationenketten eingesetzt, wobei diese Ketten in linearer oder verzweigter Form vorliegen können. Besonders bevorzugt sind die erfindungsgemäßen Hohlfasermaterialien durch Kationen- bzw. Polykationenketten, welche tertiäre und/oder quartäre Amine aufweisen, modifiziert.

Bevorzugte Anionenaustauschergruppen auf den Hohlfasermaterialien schließen Di- oder Trialkylaminoalkyl-, Di- oder Trialkylaminoaryl-, Di- oder Triarylaminoalkyl-, Di- oder Triarylaminoaryl-, Di- oder Trialkylammoniumalkyl-, Di- oder Triarylammoniumalkyl-, Di- oder Triarylammoniumaryl- und Di- oder Trialkylammoniumaryl-Reste ein. Des Weiteren sind Polymere aus positiv geladenen oder tertiäre oder quartäre Aminogruppen enthaltenden Aminosäuren, wie Polylysin, Polyarginin oder Polyhistidin oder Mischpolymere oder Derivate hiervon im Rahmen der Erfindung als Anionenaustauschermaterialien geeignet, ebenso Polyethylenimin. Besonders bevorzugt enthält die Vorrichtung ein mit Diethylaminoalkyl- bzw. Diethylaminoaryl-Resten modifiziertes Polyamid-Hohlfasermaterial, insbesondere Diethylaminoethyl-Polyamid.

Die Vielzahl der Hohlfasern/ Hohlfaserkapillaren bildet eine Hohlfasermembran aus.

Das Gehäuse der erfindungsgemäßen Vorrichtung kann als Membranmodul gesehen werden, welches in seinem Inneren eine Hohlfasermembran/ eine Vielzahl von porösen Hohlfasern aufweist. Die erfindungsgemäße Vorrichtung ist somit ähnlich einem Dialysator mit Blutkappen und Sideport aufgebaut. Die Poren der Hohlfasern weisen eine Größe von etwa 0,1 bis 0,5 µm auf, so dass lediglich das Plasma des Bluts durch die Poren strömen kann, nicht jedoch die zellulären Bestandteile/ Blutzellen. Die Hohlfasern/ die Hohlfasermembran weisen/ weist eine große innere Oberfläche auf.

Kern der vorliegenden Erfindung ist es, dass lediglich die Innenseitenoberflächen der modifizierten Hohlfasern, welche in Kontakt mit den zellulären Bestanteilen des Bluts kommen, wenn Blut die Hohlfasern durchströmt, mit einer Beschichtung/ Abdeckung beschichtet bzw. abgedeckt werden, welche die zellulären Bestandteile des Bluts nicht schädigt. Somit liegt erfindungsgemäß an den Innenseitenoberflächen der Hohlfasern keine funktionalisierte, die Noxen an sich bindende und aus dem Blut entfernende Oberfläche mehr vor. Lediglich die Poren und Außenseitenoberflächen der Hohlfasern weisen somit die funktionalisierte, die Noxen an sich bindende und aus dem Blut entfernende Oberfläche auf. Somit können die zellulären Bestandteile die Hohlfasern durchströmen ohne geschädigt zu werden. Die Noxen werden aus dem Plasma entfernt, wenn das Plasma die Poren durchströmt bzw. an den Außenseitenoberflächen der Hohlfasern entlangströmt. Die Vorrichtung der vorliegenden Erfindung stellt somit einen regioselektiven Membranadsorber bereit.

Bevorzugt ist die Beschichtung somit derart an der Innenseitenoberfläche der Hohlfasern angeordnet oder ausgebildet, dass die Innenumfangsseiten der Poren nicht oder unvollständig von der Beschichtung abgedeckt sind.

In vorteilhafter Weise ist die Beschichtung an der Innenseitenoberfläche der Hohlfasern sowohl hämokompatibel und gerinnungshemmend als auch kompatibel zu der funktionalisierten, die Noxen an sich bindenden und aus dem Blut entfernenden Oberfläche der Hohlfasern, auf welche die Beschichtung an der Innenseite der Hohlfasern aufgebracht ist. Insbesondere ist die Beschichtung/Abdeckung kompatibel zu den zellulären Bestandteilen des Bluts, damit diese beim Durchströmen durch die Vorrichtung nicht geschädigt werden. Außerdem ist die Beschichtung bzw. die durch die Beschichtung ausgebildete Substanz kompatibel zu der funktionalisierten, die Noxen an sich bindenden und aus dem Blut entfernenden ursprünglichen (Innenseiten-) Oberfläche der Hohlfasern, so dass die Beschichtung gut daran haftet. In anderen Worten wird die hämoinkompatible Oberfläche der Hohlfasern/ der Hohlfasermembran auf der Blutseite mit einer hämokompatiblen Substanz/ Beschichtung abgedeckt, die sowohl kompatibel zu der hämoinkompatiblen Oberfläche als auch sehr gut blutverträglich ist.

Ein bevorzugtes Ausführungsbeispiel ist dadurch gekennzeichnet, dass die Beschichtung auf die Innenseitenoberfläche der Hohlfasern aufgebracht wird, indem eine Lösung durch die Hohlfasern geströmt wird. In anderen Worten wird die Lösung/ Substanz auf der Blutseite durch die Hohlfasermembran/ die Hohlfasern geströmt. Bevorzugt wird dabei die funktionalisierte Oberfläche der Hohlfasern an der Innenseite der Hohlfasern entfunktionalisiert bzw. defunktionalisiert, das heißt durch die Lösung gesättigt oder abgebunden. Bevorzugt ist die Lösung/ enthält die Lösung eine gerinnungshemmende Substanz, welche sich an der Innenseitenoberfläche der Hohlfasern anbindet.

Weiter bevorzugt ist die Lösung eine negative geladene anionische Lösung, insbesondere ein gerinnungshemmendes Polyanion, bevorzugt Heparin. Dadurch kann erreicht werden, dass die grundsätzlich positiv geladene, funktionalisierte Oberfläche der Hohlfasern durch die negativ geladene Lösung gesättigt/ neutralisiert/ entladen wird. In anderen Worten wird eine Schädigung der Blutzellen in der vorliegenden Erfindung gerade dadurch vermieden/ verhindert, dass die funktionellen Gruppen an der Innenseitenoberfläche der Hohlfasern durch die Lösung abgebunden/ gesättigt werden und sich gleichzeitig eine gerinnungshemmende Substanz wie beispielsweise Heparin an die Innenseitenoberfläche der Hohlfasern anbindet.

In einem bevorzugten Ausführungsbeispiel der vorliegenden Erfindung ist die Beschichtung somit eine anionische Beschichtung. Es sind jedoch erfindungsgemäß auch andere Beschichtungen wie etwa kationische oder hydrophile Beschichtungen denkbar.

Bevorzugt sind über ein Einstellen einer Menge, einer Flussrate und vorzugsweise einer Anionenkonzentration der (anionischen) Lösung eine Beschichtung von lediglich der Innenseitenoberflächen der Hohlfasern und eine Sättigung der Innenseitenoberflächen der Hohlfasern mit der (anionischen) Lösung erreichbar und ist eine Dicke der Beschichtung variierbar.

Es hat sich herausgestellt, dass insbesondere die Parameter Menge/ Flüssigkeitsmenge/ Volumen der (anionischen) Lösung, welche in die Vorrichtung gegeben wird, sowie Flussrate der (anionischen) Lösung, mit welcher die Lösung die Vorrichtung/ die Hohlfasern durchströmt, geeignet eingestellt werden müssen. Wird eine anionische Lösung verwendet, hat auch der Parameter der Anionenkonzentration einen großen Einfluss und ist geeignet einzustellen. Dabei wirkt sich die Menge/ Flüssigkeitsmenge/ das Volumen der Lösung insbesondere auf die Sättigung der Innenseitenoberflächen mit der Lösung und die an der Innenseitenoberfläche erreichbare Dicke bzw. Schichtdicke der Beschichtung/ Abdeckung aus. In anderen Worten ist die Dicke der Abdeckung/ Beschichtung durch die Menge der die Hohlfasern durchströmenden Lösung/ Substanz steuerbar/ einstellbar (Mengensteuerung). Die Dicke der Beschichtung wird bevorzugt derart eingestellt, dass lediglich ein kleiner Teil der vorhandenen Fläche und damit der verfügbaren Kapazität verloren geht. Die Flussrate und die Anionenkonzentration wirken sich insbesondere darauf aus, dass lediglich die Innenseitenoberflächen der Hohlfasern beschichtet werden, nicht aber die Poren und Außenseitenoberflächen der Hohlfasern. Es sei an dieser Stelle angemerkt, dass sich die aktive Oberfläche für die Entfernung der Noxen im Wesentlichen in den Poren/ in der Membran befindet. Die wirksame Oberfläche in den Poren ist bevorzugt über 1500-mal größer (beispielsweise etwa 1600-mal größer) als die Innenseitenoberfläche oder die Außenseitenoberfläche der Hohlfaser. Vor diesem Hintergrund entsteht durch die Inaktivierung der Innenseitenoberfläche der Hohlfaser nur ein vernachlässigbarer Kapazitätsverlust.

Bevorzugt ist das Gehäuse auslassseitig verschlossen, insbesondere über ein Ventil, wenn die Lösung in die Hohlfasern gegeben wird. Durchströmt Blut die Vorrichtung ist das Gehäuse auslassseitig geöffnet, so dass die Vorrichtung während der Behandlung eines Patienten grundsätzlich nicht in dem sogenannten Dead-End-Verfahren betrieben wird.

In anderen Worten wird, um eine Hämokompatibilität mit (Voll-)Blut zu erreichen, zunächst der Auslass der Hohlfasern verschlossen und die Innenseite der Hohlfasern mit einer negativ geladenen Lösung durchströmt, so dass die positiv geladenen Gruppen an der Innenseite der Hohlfasern mit der negativ geladenen Lösung gesättigt werden, die Poren der Hohlfasern jedoch nicht. Dies kann über die Menge/ Flussrate/ Konzentration der durchströmenden Lösung eingestellt werden. Während einer Behandlung eines Patienten sind die Enden der Hohlfasern/ der Hohlfaserkapillaren geöffnet und die Hohlfasern werden mit Blut durchströmt. Dabei verhindern die abgebundenen funktionellen Gruppen und die gebundene gerinnungshemmende Substanz an der Innenseite der Hohlfasern eine Schädigung der Blutzellen.

Bevorzugt ist die Beschichtung während einer Behandlung eines Patienten nachdosierbar.

Weiter bevorzugt ist die Vorrichtung im Tangentialfilter-Design ausgeführt, so dass beide Enden des Gehäuses offen sind.

Ferner ist die Vorrichtung bevorzugt auch als Plasmafilter für eine Langzeitanwendung verwendbar.

Weiterhin betrifft die Erfindung ein extrakorporales Perfusionssystem mit einer Vorrichtung zur Entfernung von Noxen aus Blut wie voranstehend beschrieben. Insbesondere weist das extrakorporale Perfusionssystem ferner eine Pumpe auf, welche das Plasma des Bluts zumindest zum Teil über die Poren aus den Hohlfasern herausfördert und stromabwärts der Vorrichtung wieder dem Blut, welches die Vorrichtung durchströmt hat, zuführt.

Da die Vorrichtung eingerichtet ist, sowohl von den zellulären Bestandteilen als auch von dem Plasma des Bluts durchströmt zu werden, so dass keine Separation des Plasmas von den zellulären Bestandteilen vor dem Durchströmen der Hohlfasern mit Blut vonnöten ist, ist in dem extrakorporalen Perfusionssystem der vorliegenden Erfindung keine Plasmaseparation/ kein Plasmaseparator vonnöten.

Die Erfindung betrifft ferner ein Verfahren zur Herstellung einer Vorrichtung zur Entfernung von negativ geladenen Noxen aus Blut, insbesondere einer Vorrichtung wie voranstehend beschrieben, mit den Schritten: a) Herstellen einer Vielzahl von porösen Hohlfasern, vorzugsweise aus Kunststoff, weiter vorzugsweise aus Polyamid, Polysulfon, Polyether, Polypropylen, Polyester oder Derivaten und/oder Mischungen solcher Polymere; b) Modifizieren bzw. Vorbehandeln der Hohlfasern chemisch, vorzugsweise durch Pfropfpolymerisation, derart, dass sie eine funktionalisierte, die negativ geladenen Noxen an sich bindende und aus dem Blut entfernende positiv geladene Oberfläche aufweisen; c) Einsetzen der Vielzahl von porösen Hohlfasern in ein Gehäuse; und d) Strömen der sich in dem Gehäuse befindlichen Vielzahl von porösen Hohlfasern mit einer negativ geladenen anionischen Lösung, insbesondere ein gerinnungshemmendes Polyanion, bevorzugt Heparin (vor einem Behandlungsbeginn), so dass lediglich Poren und eine Außenseitenoberfläche der Hohlfasern die funktionalisierte Oberfläche aufweisen und an der Innenseitenoberfläche der Hohlfasern keine funktionalisierte Oberfläche mehr vorliegt; wobei die Verfahrensschritte a) bis d) in chronologischer Reihenfolge durchgeführt werden, also zuerst a), dann b), dann c) und schließlich d).

Das erfindungsgemäße Verfahren eignet sich insbesondere für eine Modifikation einer Innenbeschichtung von Hohlfasern/ Hohlfaserkapillaren.

Bevorzugt weist das Verfahren ferner den Schritt auf: e) Einstellen einer Menge und einer Flussrate der Lösung; wobei Verfahrensschritt e) vor Verfahrensschritt d) durchgeführt wird.

Weiter bevorzugt weist das Verfahren außerdem den Schritt auf: f) Verschließen des Gehäuses auslassseitig, insbesondere über ein Ventil, bevor die Lösung in die Hohlfasern gegeben wird.

Es sei angeführt, dass im Hinblick auf die Merkmale des erfindungsgemäßen Verfahrens weiterhin vollumfänglich auf die voranstehenden Ausführungen, welche die erfindungsgemäße Vorrichtung und das erfindungsgemäße extrakorporale Perfusionssystem betreffen, Bezug genommen wird. Ferner wird hinsichtlich der Verfahrensschritte a) und b) vollumfänglich auf die EP 1 602 387 A1 Bezug genommen.

### Kurzbeschreibung der Figuren

Die Erfindung wird nachfolgend mit Hilfe von Figuren weiter erläutert. Es zeigen:
- Fig. 1: eine schematische Ansicht eines erfindungsgemäßen extrakorporalen Perfusionssystems;
- Fig. 2: eine perspektivische Ansicht einer erfindungsgemäßen Vorrichtung zur Entfernung von Noxen aus Blut;
- Fig. 3: eine perspektivische Seitenansicht der erfindungsgemäßen Vorrichtung;
- Fig. 4: eine schematische Schnittansicht der erfindungsgemäßen Vorrichtung;
- Fig. 5: eine perspektivische Ansicht einer in der Vorrichtung vorgesehenen Hohlfaser;
- Fig. 6: eine schematische Ansicht der Hohlfaser;
- Fig. 7: eine schematische Schnittansicht der Hohlfaser, in welcher eine aus dem Stand der Technik bekannte Blutbehandlung veranschaulicht ist;
- Fig. 8: eine schematische Schnittansicht der Hohlfaser, in welcher eine erfindungsgemäße Blutbehandlung veranschaulicht ist; und
- Fig. 9: ein Flussdiagramm des erfindungsgemäßen Verfahrens.

### Figurenbeschreibung

Die Figuren sind lediglich schematischer Natur und dienen ausschließlich dem Verständnis der Erfindung. Gleiche Elemente sind dabei mit denselben Bezugszeichen versehen.

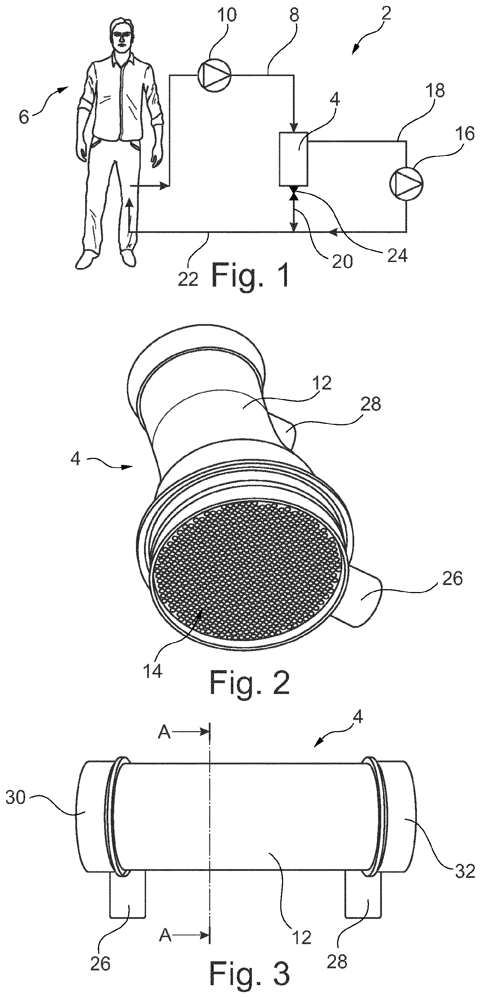

Fig. 1 zeigt eine schematische Ansicht eines erfindungsgemäßen extrakorporalen Perfusionssystems 2 mit einer Vorrichtung 4 zur Entfernung von Noxen aus Blut. Dabei wird einem Menschen 6 Blut entnommen, welches über eine erste Leitung 8 mittels einer ersten Pumpe 10 zu der Vorrichtung 4 gepumpt wird. Die Vorrichtung 4 weist, wie beispielsweise in Fig. 2 gezeigt ist, ein Gehäuse 12 und eine Vielzahl von sich innerhalb des Gehäuses 12 befindlichen Hohlfasern 14 auf. Das Blut wird dabei im Wesentlichen den Hohlfasern 14 zugeführt. Die Hohlfasern 14 sind porös, so dass das Plasma des Bluts (Blutplasma) zumindest zum Teil mittels einer zweiten Pumpe 16 aus der Vorrichtung 4 heraus in eine zweite Leitung 18 gesaugt/ gepumpt werden kann. Zumindest die zellulären Bestandteile des Bluts wie Erythrozyten, Leukozyten oder Thrombozyten verlassen die Vorrichtung 4 über eine dritte Leitung 20. Stromabwärts der Vorrichtung 4 laufen die zweite Leitung 18 und die dritte Leitung 20 wieder zusammen und das Blut wird über eine vierte Leitung 22 wieder dem Menschen 6 zugeführt. In dem erfindungsgemäßen extrakorporalen Perfusionssystem 2 wird das Blut mit allen seinen Bestandteilen, also insbesondere sowohl Plasma als auch Blutzellen, der Vorrichtung 4 zugeführt. Es ist kein separater Plasmaseparator, welcher das Plasma von den Blutzellen trennt, vonnöten. Die Vorrichtung 4 ist dabei eingerichtet, das Blut zu reinigen bzw. Noxen aus dem Blut zu entfernen. Auslassseitig der Vorrichtung 4/ anfangs der dritten Leitung 20 ist ein Absperrventil 24 vorgesehen.

Fig. 2 zeigt eine perspektivische Ansicht der erfindungsgemäßen Vorrichtung 4, welche ein Gehäuse 12 und eine Vielzahl von sich innerhalb des Gehäuses 12 befindlichen Hohlfasern 14 aufweist. Das Gehäuse 12 weist eine im Wesentlichen rohrförmige/ tubuläre/ zylindrische Form auf. An der Außenumfangsfläche des Gehäuses 12 ist nahe einer Einlassseite des Gehäuses 12 ein erster Anschluss 26 und nahe einer Auslassseite des Gehäuses 12 ein zweiter Anschluss 28 vorgesehen. Die in Fig. 1 dargestellte zweite Leitung 18 kann an dem ersten Anschluss 26 und/oder an dem zweiten Anschluss 28 angeschlossen sein, um mittels der zweiten Pumpe 16 das Plasma des Bluts aus der Vorrichtung 4 herauszufördern.

Fig. 3 zeigt eine perspektivische Seitenansicht der erfindungsgemäßen Vorrichtung 4. In der in Fig. 3 gezeigten Ansicht ist die Vorrichtung 4 einlassseitig mittels einer ersten Abdeckkappe 30 und auslassseitig mittels einer zweiten Abdeckkappe 32 abgedeckt. Die Abdeckkappen 30, 32 sind identisch ausgebildet und in ihrer Form und Größe an den runden/kreisförmigen Einlass bzw. Auslass des Gehäuses 12 angepasst.

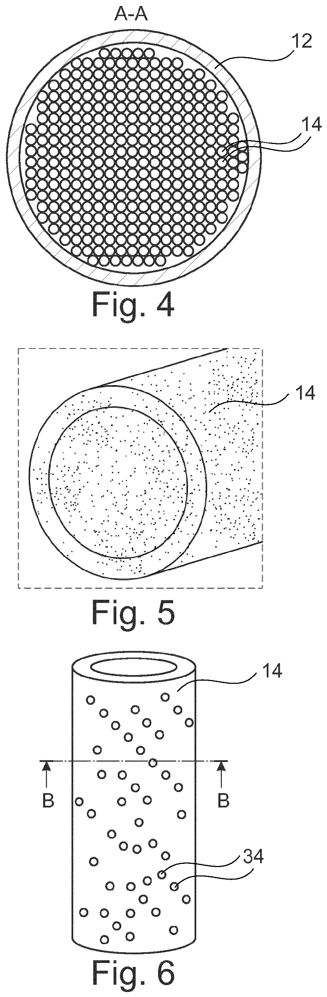

Fig. 4 zeigt eine schematische Schnittansicht der erfindungsgemäßen Vorrichtung 4, geschnitten an der in Fig. 3 dargestellten Schnittlinie A-A. In der in Fig. 4 gezeigten Ansicht sind die Hohlfasern 14 etwas vergrößert dargestellt, um die Anordnung der Hohlfasern 14 innerhalb des Gehäuses 12 besser als dies in Fig. 2 der Fall ist zu verdeutlichen. Die Vielzahl von Hohlfasern 14 erstrecken sich in einer Längsrichtung/ Axialrichtung des im Wesentlichen rohrförmigen/ zylindrischen Gehäuses 12 und füllen im Wesentlichen einen gesamten durch das Gehäuse 12 definierten Innenraum aus (vgl. auch Fig. 2). Die Gesamtheit der Hohlfasern 14 bildet eine Hohlfasermembran.

Fig. 5 zeigt eine vergrößerte perspektivische Ansicht einer einzelnen in der Vorrichtung 4 vorgesehenen Hohlfaser 14. Das Grundmaterial der Hohlfaser 14 ist bevorzugt Polyamid, auf welches Diethylaminoalkyl oder Diethylaminoaryl tentakelartig aufgepfropft ist (nicht dargestellt). Wie in Fig. 5 angedeutet ist, sind die Hohlfasern 14 porös.

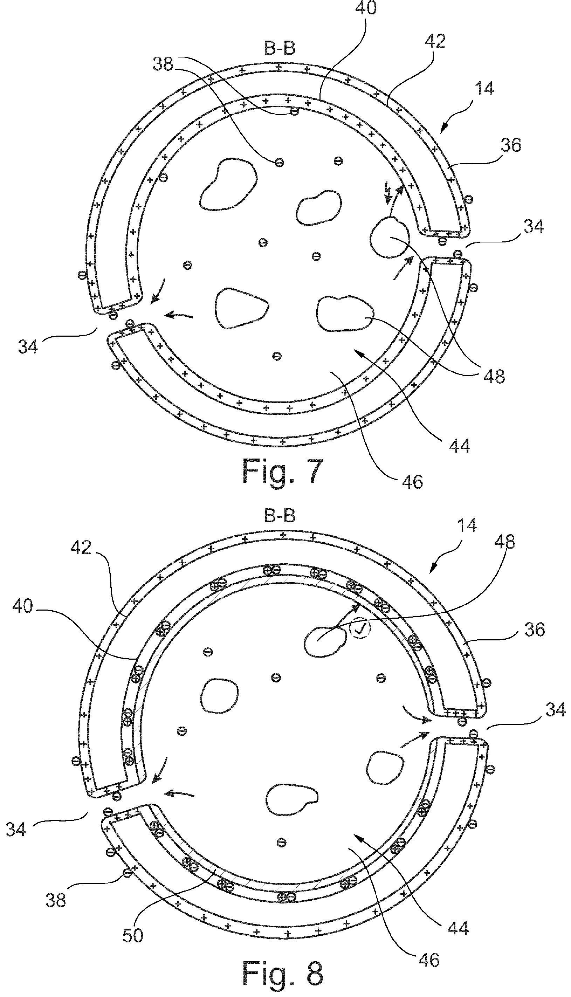

In Fig. 6 ist eine schematische Ansicht der Hohlfaser 14 dargestellt, in welcher die poröse Struktur mit Hilfe einer Vielzahl von vergrößert dargestellten Poren 34 dargestellt ist. In Fig. 7 und Fig. 8 sind Schnittansichten der in Fig. 6 dargestellten Hohlfaser 14, geschnitten an der in Fig. 6 gezeigten Schnittlinie B-B dargestellt.

Die Kernaspekte der vorliegenden Erfindung werden anhand von Fig. 7 und Fig. 8 erläutert. Dabei veranschaulichen Fig. 7 eine aus dem Stand der Technik der EP 1 602 387 A1 bekannte Blutbehandlung und Fig. 8 eine erfindungsgemäße Blutbehandlung.

Die in Fig. 7 gezeigte Hohlfaser 14 weist eine funktionalisierte Oberfläche 36 auf. Die funktionalisierte Oberfläche 36 ist positiv geladen und eingerichtet, Noxen 38, welche sich in Blut 44 befinden, an sich zu binden bzw. aus dem Blut 44 zu entfernen. Die funktionalisierte Oberfläche 36 ist sowohl an einer Innenseitenoberfläche 40 der Hohlfaser 14 als auch an einer Außenseitenoberfläche 42 der Hohlfaser 14 als auch im Bereich der Poren 34 vorgesehen. Die funktionalisierte Oberfläche 36 wird durch eine chemische Modifikation, insbesondere über eine Pfropfpolymerisation, erzeugt.

Durchströmt nun Blut 44, welches Blutplasma 46 und Blutzellen 48 aufweist, die in Fig. 7 dargestellte Hohlfaser 14, werden die sich in dem Blut 44 bzw. insbesondere in dem Blutplasma 46 befindlichen negativ geladenen Noxen 38 sowohl an der Innenseitenoberfläche 40 als auch an der Außenseitenoberfläche 42 als auch im Bereich der Poren 34 an die positiv geladene (Oberfläche der) Hohlfaser 14 gebunden und somit aus dem Blut entfernt. Die Größe bzw. der Durchmesser der Poren 34 ist derart ausgebildet, dass die Blutzellen 48 nicht durch die Poren 34 strömen können. Gelangen die in Fig. 7 gezeigten Blutzellen 48 in Kontakt mit der funktionalisierten Oberfläche 36 werden die Blutzellen 48 geschädigt/ zerstört, wie in Fig. 7 durch einen Blitz angedeutet ist. Daher müssen im Stand der Technik die Blutzellen 48 von dem Blutplasma 46 getrennt werden, so dass die Blutzellen 48 nicht in die Vorrichtung 4 bzw. in die Hohlfasern 14 gelangen.

Gemäß der vorliegenden Erfindung ist die Innenseitenoberfläche 40 der Hohlfasern 14 weiterhin mit einer hämokompatiblen und gerinnungshemmenden Beschichtung 50 versehen (siehe Fig. 8). Die Beschichtung 50 wird aufgebracht, indem eine negativ geladene anionische Lösung (beispielsweise ein gerinnungshemmendes Polyanion wie etwa Heparin) durch die Hohlfasern 14 geströmt wird (vor der dargestellten Blutbehandlung). Dadurch wird erreicht, dass zum einen die funktionalisierte, positiv geladene Oberfläche 36 an der Innenseitenoberfläche 40 der Hohlfasern 14 durch die negativ geladene anionische Lösung abgebunden/ entladen/ neutralisiert wird, wie in Fig. 8 durch die aneinanderhängenden positiven und negativen, sich entsprechend neutralisierenden, Ladungen an der Innenseitenoberfläche 40 der Hohlfasern 14 veranschaulicht ist. Zum anderen bindet sich eine Beschichtung 50 an die (zuvor) funktionalisierte Oberfläche 36 an. Die Beschichtung 50 ist hämokompatibel und gerinnungshemmend, so dass die die Hohlfasern 14 durchströmenden Blutzellen 48 nicht geschädigt werden, wenn sie auf die Innenseitenoberfläche 40 treffen (in Fig. 8 durch einen Haken angedeutet). Die Beschichtung 50 ist kompatibel zu der funktionalisierten Oberfläche 36 und haftet an dieser an.

Durchströmt nun Blut 44, welches Blutplasma 46 und Blutzellen 48 aufweist, die in Fig. 8 dargestellte Hohlfaser 14, werden die sich in dem Blut 44 bzw. insbesondere in dem Blutplasma 46 befindlichen negativ geladenen Noxen 38 lediglich an der Außenseitenoberfläche 42 und im Bereich der Poren 34 an die positiv geladene (Oberfläche der) Hohlfaser 14 gebunden und somit aus dem Blut entfernt. An der Innenseitenoberfläche 40 der Hohlfaser 14 werden keine Noxen 38 gebunden, und wie bereits ausgeführt dafür die Blutzellen 48 auch nicht geschädigt. In der erfindungsgemäßen Vorrichtung 4 ist es somit nicht notwendig, die Blutzellen 48 von dem Blutplasma 46 vor der Vorrichtung 4 zu trennen.

Über ein Einstellen einer Flussrate und einer Anionenkonzentration der anionischen Lösung, welche vor der dargestellten Blutbehandlung durch die Hohlfaser 14 geströmt wird, kann erreicht werden, dass lediglich die Innenseitenoberflächen 40 der Hohlfasern 14, nicht jedoch die Poren 34 und die Außenseitenoberflächen 42 der Hohlfasern 14 beschichtet werden. Dies wird insbesondere dadurch erreicht, dass die Flussrate auf einen geringen Wert und die Anionenkonzentration auf einen hohen Wert (viskosere anionische Lösung) eingestellt wird. Über ein Einstellen der Menge der anionischen Lösung kann eine Sättigung der Innenseitenoberflächen 40 der Hohlfasern 14 und eine Dicke der Beschichtung 50 eingestellt werden. Hier gilt, dass die Beschichtung 50 umso dicker wird, je größer die in die Hohlfasern 14 gegebene Menge/ Flüssigkeitsmenge ist.

Das in Fig. 1 dargestellte Absperrventil 24 ist geschlossen, wenn die anionische Lösung in die Vorrichtung 4 bzw. in die Vielzahl von Hohlfasern 14 gegeben wird.

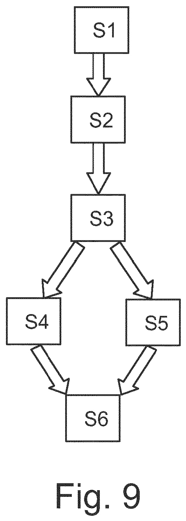

Fig. 9 veranschaulicht ein Flussdiagramm des erfindungsgemäßen Verfahrens. Gemäß dem erfindungsgemäßen Verfahren erfolgt zunächst in Schritt S1 ein Herstellen einer Vielzahl von porösen Hohlfasern 14. Anschließend erfolgt in Schritt S2 ein Modifizieren/ Vorbehandeln der Hohlfasern 14 derart, dass sie eine funktionalisierte, die Noxen an sich bindende und aus dem Blut 44 entfernende Oberfläche 36 aufweisen. Dann wird in einem Schritt S3 die Vielzahl von porösen Hohlfasern 14 in ein Gehäuse 12 eingesetzt. In einem Schritt S4 wird das Gehäuse 12 auslassseitig über ein Ventil (das Absperrventil 24) verschlossen und parallel dazu werden in einem Schritt S5 eine Menge, eine Flussrate sowie eine Anionenkonzentration einer anionischen Lösung eingestellt. Schließlich werden die sich in dem Gehäuse 12 befindlichen Hohlfasern 14 mit der anionischen Lösung in einem Schritt S6 geströmt/ durchströmt.

### Bezugszeichenliste

- 2: extrakorporales Perfusionssystem
- 4: Vorrichtung
- 6: Mensch
- 8: erste Leitung
- 10: erste Pumpe
- 12: Gehäuse
- 14: Hohlfaser
- 16: zweite Pumpe
- 18: zweite Leitung
- 20: dritte Leitung
- 22: vierte Leitung
- 24: Absperrventil
- 26: erster Anschluss
- 28: zweiter Anschluss
- 30: erste Abdeckkappe
- 32: zweite Abdeckkappe
- 34: Poren
- 36: funktionalisierte Oberfläche
- 38: Noxen
- 40: Innenseitenoberfläche
- 42: Außenseitenoberfläche
- 44: Blut
- 46: Blutplasma
- 48: Blutzellen
- 50: (hämokompatible und gerinnungshemmende) Beschichtung

## Patentansprüche

1. Vorrichtung (4) zur Entfernung von negativ geladenen Noxen (38) aus Blut (44), welches Plasma (46) und zelluläre Bestandteile (48) aufweist, in einem extrakorporalen Perfusionssystem (2), mit:
einem Gehäuse (12) und einer Vielzahl von innerhalb des Gehäuses (12) vorgesehenen Hohlfasern (14), welche konfiguriert sind, mit dem Blut (44) durchströmt zu werden, wobei
die Hohlfasern (14) jeweils eine Vielzahl von Poren (34) aufweisen, welche derart ausgebildet sind, dass das Plasma (46) des Bluts (44) durch die Poren (34) von einer Innenseite der Hohlfasern (14) zu einer Außenseite der Hohlfasern (14) strömen kann, und wobei
die Hohlfasern (14) derart chemisch modifiziert bzw. vorbehandelt sind, dass sie eine funktionalisierte, die negativ geladenen Noxen (38) an sich bindende und aus dem Blut entfernende positiv geladene Oberfläche (36) aufweisen, wobei
ausschließlich eine Innenseitenoberfläche (40) der Hohlfasern weiterhin zur Vermeidung einer Schädigung der zellulären Bestandteile (48) des Bluts (44) beim Strömen des Bluts (44) durch die Hohlfasern (14) mit einer hämokompatiblen und gerinnungshemmenden Beschichtung (50) versehen ist, so dass lediglich die Poren (34) und eine Außenseitenoberfläche (42) der Hohlfasern (14) die funktionalisierte Oberfläche (36) aufweisen und an der Innenseitenoberfläche (40) der Hohlfasern (14) keine funktionalisierte Oberfläche (36) mehr vorliegt.

2. Vorrichtung (4) nach Anspruch 1, **dadurch gekennzeichnet, dass** die Beschichtung (50) derart an der Innenseitenoberfläche (40) der Hohlfasern (14) angeordnet oder ausgebildet ist, dass die Innenumfangsseiten der Poren (34) nicht oder unvollständig von der Beschichtung (50) abgedeckt sind.

3. Vorrichtung (4) nach Anspruch 1 oder 2, **dadurch gekennzeichnet, dass** die Beschichtung (50) an der Innenseitenoberfläche (40) der Hohlfasern (14) sowohl hämokompatibel und gerinnungshemmend als auch kompatibel zu der funktionalisierten, die Noxen (38) an sich bindenden und aus dem Blut entfernenden Oberfläche (36) der Hohlfasern (14) ist, auf welche die Beschichtung (50) an der Innenseite der Hohlfasern (14) aufgebracht ist.

4. Vorrichtung (4) nach einem der Ansprüche 1 bis 3, **dadurch gekennzeichnet, dass** die Beschichtung (50) auf die Innenseitenoberfläche (40) der Hohlfasern (14) durch Strömen einer Lösung durch die Hohlfasern (14) aufgebracht ist.

5. Vorrichtung (4) nach Anspruch 4, **dadurch gekennzeichnet, dass** die Lösung eine negative geladene anionische Lösung, insbesondere ein gerinnungshemmendes Polyanion, bevorzugt Heparin, ist.

6. Vorrichtung (4) nach Anspruch 4 oder 5, **dadurch gekennzeichnet, dass** über ein Einstellen einer Menge, einer Flussrate und vorzugsweise einer Anionenkonzentration der Lösung ein Beschichten von lediglich der Innenseitenoberflächen (40) der Hohlfasern (14) und eine Sättigung der Innenseitenoberflächen (40) der Hohlfasern (14) mit der Lösung erreichbar sind und eine Dicke der Beschichtung (50) variierbar ist.

7. Vorrichtung (4) nach einem der Ansprüche 4 bis 6, **dadurch gekennzeichnet, dass** das Gehäuse (12) auslassseitig verschlossen ist, insbesondere über ein Ventil (24), wenn die Lösung in die Hohlfasern (14) gegeben wird.

8. Extrakorporales Perfusionssystem (2) mit einer Vorrichtung (4) zur Entfernung von Noxen (38) aus Blut (44) nach einem der vorhergehenden Ansprüche und einer Pumpe (16), welche das Plasma (46) des Bluts (44) über die Poren (34) aus den Hohlfasern (14) herausfördert und stromabwärts der Vorrichtung (4) wieder dem Blut (44), welches die Vorrichtung (4) durchströmt hat, zuführt.

9. Verfahren zur Herstellung einer Vorrichtung (4) zur Entfernung von negativ geladenen Noxen (38) aus Blut (44) nach einem der Ansprüche 1 bis 7, mit den Schritten:
a) Herstellen einer Vielzahl von porösen Hohlfasern (14);
b) Modifizieren bzw. Vorbehandeln der Hohlfasern (14) chemisch derart, dass sie eine funktionalisierte, die negativ geladenen Noxen (38) an sich bindende und aus dem Blut (44) entfernende positiv geladene Oberfläche (36) aufweisen;
c) Einsetzen der Vielzahl von porösen Hohlfasern (14) in ein Gehäuse (12); und
d) Strömen der sich in dem Gehäuse (12) befindlichen Vielzahl von porösen Hohlfasern (14) mit einer negativ geladenen anionischen Lösung, so dass lediglich Poren (34) und eine Außenseitenoberfläche (42) der Hohlfasern (14) die funktionalisierte Oberfläche (36) aufweisen und an der Innenseitenoberfläche (40) der Hohlfasern (14) keine funktionalisierte Oberfläche (36) mehr vorliegt;
wobei die Verfahrensschritte a) bis d) in chronologischer Reihenfolge durchgeführt werden.

10. Verfahren nach Anspruch 9, ferner mit dem Schritt:
e) Einstellen einer Menge und einer Flussrate der Lösung; wobei Verfahrensschritt e) vor Verfahrensschritt d) durchgeführt wird.

11. Verfahren nach Anspruch 9 oder 10, ferner mit dem Schritt:
f) Verschließen des Gehäuses (12) auslassseitig, insbesondere über ein Ventil (24), bevor die Lösung in die Hohlfasern (14) gegeben wird.

## Claims

1. A device (4) for removing negatively charged noxae (38) from blood (44), which comprises plasma (46) and cellular components (48), in an extracorporeal perfusion system (2), comprising:
a housing (12) and a plurality of hollow fibers (14) provided inside the housing (12) and configured to be perfused by the blood (44), wherein
the hollow fibers (14) each have a plurality of pores (34) configured such that the plasma (46) of the blood (44) can flow through the pores (34) from an inside of the hollow fibers (14) to an outside of the hollow fibers (14), and wherein
the hollow fibers (14) are modified or pretreated chemically in such a way that they have a functionalized, positively charged surface (36) which binds the negatively charged noxae (38) to itself and removes them from the blood, wherein
exclusively an inside surface (40) of the hollow fibers is further provided with a hemocompatible and anticoagulant coating (50) to prevent damage of the cellular components (48) of the blood (44) when the blood (44) flows through the hollow fibers (14), so that merely the pores (34) and an outside surface (42) of the hollow fibers (14) have the functionalized surface (36) and on an inside surface (40) of the hollow fibers (14), a functionalized surface (36) is no longer provided.

2. The device (4) according to claim 1, **characterized in that** the coating (50) is arranged or formed on the inside surface (40) of the hollow fibers (14) in such a way that the inner circumferential sides of the pores (34) are not covered or are incompletely covered by the coating (50).

3. The device (4) according to claim 1 or 2, **characterized in that** the coating (50) on the inside surface (40) of the hollow fibers (14) is both hemocompatible and anticoagulant as well as compatible with the functionalized surface (36) of the hollow fibers (14) which binds the noxae (38) to itself and removes them from the blood and to which the coating (50) is applied on the inside of the hollow fibers (14).

4. The device (4) according to any of claims 1 to 3, **characterized in that** the coating (50) is applied to the inside surface (40) of the hollow fibers (14) by causing a solution to flow through the hollow fibers (14).

5. The device (4) according to claim 4, **characterized in that** the solution is a negatively charged anionic solution, in particular an anticoagulant polyanion, preferably heparin.

6. The device (4) according to claim 4 or 5, **characterized in that,** by adjusting a quantity, a flow rate and preferably an anion concentration of the solution, a coating of only the inside surfaces (40) of the hollow fibers (14) and a saturation of the inside surfaces (40) of the hollow fibers (14) with the solution can be achieved and a thickness of the coating (50) can be varied.

7. The device (4) according to any of claims 4 to 6, **characterized in that** the housing (12) is closed on the outlet side, in particular via a valve (24), when the solution is introduced into the hollow fibers (14).

8. An extracorporeal perfusion system (2) comprising a device (4) for removing noxae (38) from blood (44) according to any of the preceding claims, and a pump (16) which conveys the plasma (46) of the blood (44) out of the hollow fibers (14) via the pores and feeds it back, downstream of the device (4), to the blood (44) which has flowed through the device (4).

9. A method for producing a device (4) for removing negatively charged noxae (38) from blood (44) according to any of preceding claims 1 to 7, comprising the steps of:
a) producing a plurality of porous hollow fibers (14);
b) modifying or pretreating the hollow fibers (14) chemically in such a way that they have a functionalized, positively charged surface (36) which binds the noxae (38) to itself and removes them from the blood (44);
c) introducing said plurality of porous hollow fibers (14) into a housing (12); and
d) causing a negatively charged anionic solution to flow through the plurality of porous hollow fibers (14) located in the housing (12) so that merely pores (34) and an outside surface (42) of the hollow fibers (14) have the functionalized surface (36), and on an inside surface (40) of the hollow fibers (14), a functionalized surface (36) is no longer provided;
the method steps a) to d) being carried out in chronological order.

10. The method according to claim 9, further comprising the step of:
e) adjusting a quantity and a flow rate of the solution; wherein method step e) is carried out before method step d).

11. The method according to claim 9 or 10, further comprising the step of:
f) closing the housing (12) on the outlet side, in particular via a valve (24), before the solution is introduced into the hollow fibers (14).

## Revendications

1. Dispositif (4) d'élimination du sang (44) de toxines chargées négativement (38), sang qui présente du plasma (46) et des composants cellulaires (48), dans un système de perfusion extracorporel (2), avec :
un boîtier (12) et une pluralité de fibres creuses (14) prévues à l'intérieur du boîtier (12), qui sont configurées pour être traversées par le sang (44), dans lequel
les fibres creuses (14) présentent respectivement une pluralité de pores (34) qui sont formés de sorte que le plasma (46) du sang (44) peut traverser les pores (34) depuis un côté intérieur des fibres creuses (14) vers un côté extérieur des fibres creuses (14), et dans lequel
les fibres creuses (14) sont modifiées chimiquement ou prétraitées de sorte qu'elles présentent une surface fonctionnalisée chargée positivement (36) se liant à des toxines chargées négativement (38) et les éliminant du sang, dans lequel
seule une surface de côté intérieur (40) des fibres creuses est en outre dotée d'un revêtement hémocompatible et anticoagulant (50) pour éviter d'endommager les composants cellulaires (48) du sang (44) lorsque le sang (44) traverse les fibres creuses (14), de sorte que seuls les pores (34) et une surface de côté extérieur (42) des fibres creuses (14) présentent la surface fonctionnalisée (36) et qu'une surface fonctionnalisée (36) sur la surface de côté intérieur (40) des fibres creuses (14) ne soit plus présente.

2. Dispositif (4) selon la revendication 1, **caractérisé en ce que** le revêtement (50) est disposé ou formé au niveau de la surface de côté intérieur (40) des fibres creuses (14) de sorte que les côtés de périphérie intérieure des pores (34) ne soient pas ou soient incomplètement recouverts par le revêtement (50).

3. Dispositif (4) selon la revendication 1 ou 2, **caractérisé en ce que** le revêtement (50) au niveau de la surface de côté intérieur (40) des fibres creuses (14) est aussi bien hémocompatible et anticoagulant que compatible avec la surface fonctionnalisée (36) des fibres creuses (14) se liant à des toxines (38) et les éliminant du sang, surface sur laquelle le revêtement (50) est appliqué sur le côté intérieur des fibres creuses (14).

4. Dispositif (4) selon l'une quelconque des revendications 1 à 3, **caractérisé en ce que** le revêtement (50) est appliqué sur la surface de côté intérieur (40) des fibres creuses (14) par écoulement d'une solution à travers les fibres creuses (14).

5. Dispositif (4) selon la revendication 4, **caractérisé en ce que** la solution est une solution anionique chargée négativement, en particulier un polyanion anticoagulant, de préférence de l'héparine.

6. Dispositif (4) selon la revendication 4 ou 5, **caractérisé en ce que**, en définissant une quantité, un débit et de préférence une concentration en anions de la solution, il est possible d'obtenir un revêtement uniquement sur les surfaces de côté intérieur (40) des fibres creuses (14) et une saturation des surfaces de côté intérieur (40) des fibres creuses (14) avec la solution, et une épaisseur du revêtement (50) est variable.

7. Dispositif (4) selon l'une quelconque des revendications 4 à 6, **caractérisé en ce que** le boîtier (12) est fermé côté sortie, en particulier par l'intermédiaire d'une vanne (24), lorsque la solution est ajoutée dans les fibres creuses (14).

8. Système de perfusion extracorporel (2) avec un dispositif (4) d'élimination de toxines (38) du sang (44) selon l'une quelconque des revendications précédentes et une pompe (16) qui extrait des fibres creuses (14) le plasma (46) du sang (44) par l'intermédiaire des pores (34) et le réintroduit en aval du dispositif (4) dans le sang (44) qui a traversé le dispositif (4).

9. Procédé de fabrication d'un dispositif (4) d'élimination de toxines chargées négativement (38) du sang (44) selon l'une quelconque des revendications 1 à 7, avec les étapes consistant à :
a) fabriquer une pluralité de fibres creuses poreuses (14) ;
b) modifier ou prétraiter les fibres creuses (14) chimiquement, de sorte qu'elles présentent une surface fonctionnalisée chargée positivement (36) se liant à des toxines chargées négativement (38) et les éliminant du sang (44) ;
c) introduire la pluralité de fibres creuses poreuses (14) dans un boîtier (12) ; et
d) faire s'écouler une solution anionique chargée négativement dans la pluralité de fibres creuses poreuses (14) se trouvant dans le boîtier (12), de sorte que seuls les pores (34) et une surface de côté extérieur (42) des fibres creuses (14) présentent la surface fonctionnalisée (36) et qu'aucune surface fonctionnalisée (36) ne soit plus présente sur la surface de côté intérieur (40) des fibres creuses (14).
dans lequel les étapes a) à d) du procédé sont mises en œuvre dans l'ordre chronologique.

10. Procédé selon la revendication 9, avec en outre l'étape consistant à :
e) définir une quantité et un débit de la solution ; dans lequel l'étape e) du procédé est mise en œuvre avant l'étape d) du procédé.

11. Procédé selon la revendication 9 ou 10, avec en outre l'étape consistant à :
f) fermer le boîtier (12) côté sortie, en particulier par l'intermédiaire d'une vanne (24), avant que la solution ne soit ajoutée dans les fibres creuses (14).
